# EUROPEAN PATENT APPLICATION

(11) **EP 3 975 190 A1**
(43) Date of publication of application: **30.03.2022**
(21) Application number: 19929312.7
(22) Date of filing: 23.05.2019
(51) Int. Cl.: G16B 30/00, C12Q 1/6886, G16H 20/70, G16H 50/30

(54) **METHOD FOR DISCOVERING MARKER FOR PREDICTING RISK OF DEPRESSION OR SUICIDE USING MULTI-OMICS ANALYSIS, MARKER FOR PREDICTING RISK OF DEPRESSION OR SUICIDE, AND METHOD FOR PREDICTING RISK OF DEPRESSION OR SUICIDE USING MULTI-OMICS ANALYSIS**

(71) Applicant: Ulsan National Institute of Science and Technology (UNIST), Eonyang-eup Ulju-gun Ulsan 44919 (KR)
(72) Inventor: LEE, Se Min, Ulju-gun, Ulsan 44919 (KR); BHAK, Jong Hwa, Ulju-gun, Ulsan 44919 (KR); JEONG, Hyoung Oh, Ulju-gun, Ulsan 44919 (KR); BHAK, Young June, Ulju-gun, Ulsan 44919 (KR); KIM, Byung Chul, Suwon-si Gyeonggi-do 16713 (KR); CHO, Yun Sung, Yongin-si Gyeonggi-do 16953 (KR)
(74) Representative: Bosia, Alessandra
(86) International application number: PCT/KR2019/006160
(87) International publication number: WO 2020/235721

(57) **Abstract**

The present invention relates to a method of discovering a marker for predicting a risk of depression or suicide using multi-omics analysis and machine learning, and a marker for predicting a risk of depression or suicide, discovered by the method. According to the method for discovering a marker for predicting a risk of depression or suicide, the marker for predicting the risk of depression or suicide may be discovered with high accuracy and reliability, and the risk of depression or suicide can be diagnosed and prevented at an early stage through genetic testing.

## Description

### TECHNICAL FIELD

The present invention relates to a method for discovering a marker for predicting a risk of depression or suicide using multi-omics analysis, a marker for predicting the risk of depression or suicide, and a method for predicting the risk of depression or suicide using multi-omics analysis.

### BACKGROUND ART

Currently, the observed suicide rate in Korea is the highest among OECD countries. According to a recent survey, among the causes of death of Koreans, suicide ranks next to cancer, cerebrovascular disease, and heart disease, and has been steadily increasing over the past few years. Accordingly, in the related field, the increasing suicide rate in Korea is recognized as a serious social problem, and efforts are being made to predict the suicide rate. However, the current research for suicide prediction considers only simple and fragmentary factors which affect suicide, such as unemployment rates or temperatures, and thus the reliability of the prediction results is low.

Since suicide is a violation of the human obsession with survival, psychological or social etiological theories have been supported as main causes of suicide. However, in the 21st century, it is increasingly being elucidated that genetic factors are a main cause of suicide. By noting that in all races, the suicide rate is as high as about 1% in common and this suicide rate has stayed constant, evolutionary geneticists emphasize that suicide is a genetically evolved psychopathology in that depressive symptoms are also traits acquired through evolution, and depression is clearly linked with suicide. Based on such basic perspectives, evidence for genetic factors of suicidal behavior have been provided through family, twin, and adoption studies. Some twin studies suggest that about 45% of the occurrence of suicidal ideation and suicidal behavior are caused by genetic factors. In particular, in cases of fatal suicide attempts, genetic factors are estimated to be up to 55%. Family studies have found that the inheritance of suicidal behavior is independent of the psychopathological inheritance associated with suicidal behavior. In other words, familial inheritance of stress, such as mental illness, is not related to familial inheritance of predisposition to suicidal behavior. These facts suggest that there are genetic factors associated with the predisposition to suicidal behavior.

Currently, meaningful genetic predictors of suicidal behaviors are insufficient. Therefore, there is a need in the art for diagnostic assays and tests to identify subjects at risk of suicide. Accordingly, in the present invention, proposed is a method of predicting a suicide rate with high reliability in consideration of more practical factors that affect suicide.

### DESCRIPTION OF EMBODIMENTS

### TECHNICAL PROBLEM

One aspect provides a method for discovering a marker for predicting a risk of depression or suicide using multi-omics analysis.

Another aspect provides a marker for predicting a risk of depression or suicide.

Another aspect provides a method for predicting a risk of depression or suicide using multi-omics analysis.

### SOLUTION TO PROBLEM

Since various modifications can be applied to the present invention and various embodiments can be provided, specific embodiments are illustrated in the drawings and described in the detailed description. Effects and features of the present invention, and methods of achieving the same, will become apparent with reference to the embodiments described below in detail in conjunction with the drawings. However, the present invention is not limited to the following embodiments and may be implemented in various forms.

In the following embodiments, the terms first, second, etc. are not intended to be limiting but are only used to distinguish one element component, from another.

In the following embodiments, the singular forms are intended to include the plural forms, unless the context clearly indicates otherwise.

In the following embodiments, the terms "comprises" and/or "comprising," when used in this specification, specify the presence of stated features, and/or components, but do not preclude the presence or addition of one or more other features, and/or components.

When a certain embodiment may be implemented otherwise, a particular process may be performed in a different order than described herein. For example, two processes described in succession may, in fact, be executed substantially concurrently or may sometimes be executed in the reverse order than described herein.

In the drawings, for the sake of convenient explanation, the size of each component will be exaggerated or reduced. For example, for brevity and clarity, the size and thickness of each component appearing on each drawing are shown in an arbitrary manner, and the present disclosure is not so limited.

One aspect provides a method for discovering a maker for predicting a risk of depression or suicide, the method comprising the steps of: acquiring multi-omics data for a plurality of individuals having depression, a plurality of individuals who have attempted suicide, or a plurality of individuals committing suicide, and data regarding whether or not there is depression, suicide attempt or suicide completion; generating a test model by performing machine learning on the input data for learning, processed from the multi-omics data, and the output data for learning, processed from the data regarding whether or not there is depression, suicide attempt or suicide completion; calculating the degree of predicting the risk of depression or suicide by applying the input data for learning and the output data for learning to the test model; and selecting the multi-omics data of which the prediction degree is equal to or greater than a predefined reference value.

In one embodiment, the multi-omics data may include methylation-related data or genome data.

In one embodiment, the methylation marker data or the genome data may include a change in the measured methylation level or the measured gene expression level, compared to the methylation level or the gene expression level of a comparative control group, respectively.

The comparative control group may include normal individuals, individuals who have attempted suicide, individuals committing suicide, or individuals having depression. For example, multi-omics data between patients having depression and individuals who have attempted suicide can be compared, and this is called a binary classifier model.

In one embodiment, the method of predicting a risk of depression or suicide may use machine learning.

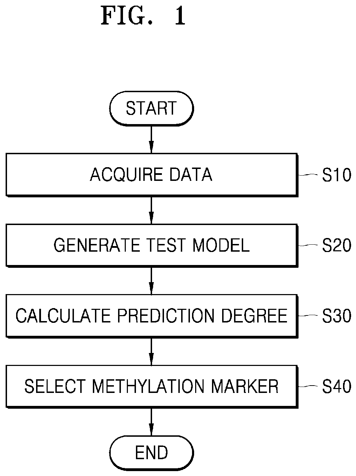

Referring to FIG. 1, a step (S10) is performed, in which multi-omics data for a plurality of individuals having depression, a plurality of individuals who have attempted suicide, or a plurality of individuals committing suicide, and data regarding whether or not there is depression, suicide attempt or suicide completion, are acquired.

The methylation-related data may refer to whether or not methylation occurs in a specific region or a specific position in the chromosome of an individual, the degree of methylation, or the ratio of methylated sequences. Whether or not methylation occurs at a specific region or at a specific position in the chromosome can be used interchangeably with the methylated site. Nucleotide methylation refers to a phenomenon in which a change in the gene expression mechanism occurs due to obtained modifications, such as DNA methylation, without accompanying changes in the nucleotide sequence. DNA methylation is involved in the inhibition of gene expression. Methylation may occur in the cytosine of the CpG dinucleotide sequence of genomic DNA. CpG sequences exist sporadically in the genome, but, specifically, methylation can occur in regions called CpG islands. Methylation of CpG islands generally inhibits chromatin aggregation and gene transcription. Genetically, DNA methylation can cause significant differences in individuals. Therefore, whether or not methylation occurs at a specific position in the chromosome can be used as an indicator for predicting the risk of depression or suicide in an individual.

As a result of sequencing in the chromosome of an individual, the methylation-related data may include records related to DNA methylation in the genome of an individual, such as the position of a methylated nucleotide in the chromosome, a gene related to the position of a methylated nucleotide in the chromosome, and the like.

After the methylation marker data are divided into a risk group (Case) including individuals having depression or individuals who have attempted or committed suicide, and a control group including normal individuals not having depression or not having attempted or committed suicide (Control), the measured methylation levels of the risk group and the normal individuals are compared. Then, the methylation-related data in which a difference in the measured methylation level is greater than 0.01 beta value and the Benjamini-Hochberg adjusted P value is less than 0.05 may be identified as a marker for predicting the risk of depression or suicide.

After the genome data are divided into a risk group (Case) including individuals having depression or individuals who have attempted or committed suicide, and a control group including normal individuals not having depression or not having attempted or committed suicide (Control), the measured gene expression levels of the risk group and the normal individuals are compared. Then, the genome data in which a difference in the measured gene expression level is 1.2 times or more and the Benjamini-Hochberg adjusted P value is less than 0.05 may be identified as a marker for predicting the risk of depression or suicide.

The suicide refers to a case in which medical treatment is required by acting with the intention of causing one's own death, and the result is a suicide attempt or suicide completion. The depression (depressive disorder) means a depressive mood or loss of interest or pleasure in most activities, which lasts for more than a certain period of time, such as changes in sleep, changes in appetite and weight, agitation, retardation, fatigue, feelings of worthlessness or guilt, and decreased ability to think and concentrate.

The data regarding whether or not there is depression, suicide attempt or suicide completion may mean, but is not limited to, a past or present pathological record of depressive disorder, a suicide attempt experience, or death due to suicide completion.

The methylation-related data and the data regarding whether or not there is depression, suicide attempt or suicide completion may be acquired from individuals from one or more hospitals or local areas. The methylation-related data may be acquired by performing a known method for confirming methylation of a genome or DNA, and the data regarding whether or not there is depression, suicide attempt or suicide completion may be obtained from an individual's questionnaire or survey result, but is limited thereto.

The individual means a subject for predicting the risk of depression or suicide. The individual may include a vertebrate, a mammal, or a human (Homo sapiens). For example, the human may be Korean.

The step of acquiring the data may include adding missing data (NaN) by using a k-nearest neighbor algorithm (knn).

Thereafter, a step (S20) is performed, in which a test model is generated by performing machine learning on the input data for learning, processed from the methylation-related data and the output data for learning, processed from the data regarding whether or not there is depression, suicide attempt or suicide completion.

Multi-omics analysis means a holistic and integrated analysis of various data generated at various molecular levels, such as genome, tranome, proteome, metabolome, epigenome, and lipodome. In multi-omics, large-scale information is produced, and thus bioinformatics techniques can be utilized.

Machine learning, which is a type of artificial intelligence, allows computers to learn on their own through given data. Machine learning includes functions and generalization for data representation and evaluation thereof. Generalization means that the current model is applied to new data.

The step of generating the test model may include obtaining a correlation between the input data for learning, processed from the multi-omics data generated by the machine learning technique and the output data for learning, processed from the data regarding whether or not there is depression, suicide attempt or suicide completion, corresponding to the multi-omics data, that is, mapping information of both data. Data for learning may include input data for learning and output data for learning.

The "input data for learning" is data used for machine learning, and may be acquired by processing multi-omics data for a plurality of individuals having depression, a plurality of individuals who have attempted suicide, or a plurality of individuals committing suicide. For example, among the above-described methylation-related data, the values that can be classified, such as a chromosome number, the position of a nucleotide in the chromosome where methylation occurs, the degree of methylation, or the ratio of methylated sequences, may be labeled to then be converted into one mathematical value.

The "output data for learning" means data that is compared with the value output through the test model or the result value of the method for predicting the risk of the depressive disorder or suicide using the same. The output data for learning may be processed and obtained from the data regarding whether or not there is depression, suicide attempt or suicide completion. For example, the "output data for learning" may be data indicating a pathological record of being diagnosed with depressive disorder at any time in the past or in the present, an experience of a suicide attempt, or death due to suicide completion. For example, if a test model is machine-learned to predict whether or not depressive disorder, suicide attempt, or suicide completion will occur at any point in the future, the "output data for learning" may be binary data expressed as 1 for a case in which there is depression or suicide attempt or suicide completion, or expressed as 0 for a case in which there is no depressive disorder or suicide attempt or suicide completion.

Through this process, multi-omics data and data regarding whether or not there is depression, suicide attempt, or suicide completion can be mathematically processed to obtain input data for learning and output data for learning.

"Test model" means an input/output function that analyzes the correlation between the input data for learning and the output data for learning and diagnose depressive disorder or predicts suicide attempt, or death due to suicide completion at any point in the past, present, or future. In this case, the test model can output a value close to 0 or 1, and the closer to 0 or smaller the output value is, the higher the probability that there would be no depressive disorder, no suicide attempt or no suicide completion, while the closer to 1 or larger the output value is, the greater the higher the probability that there would be diagnosis of depressive disorder, suicide attempt or death due to suicide completion. Therefore, the output value can be interpreted as an index indicating "depressive disorder, suicide attempt or suicide completion".

After the test model generation step (S20), based on the prediction result of the test model, a step (S30) is performed, in which the degree of predicting the risk of depression or suicide is calculated by applying the input data for learning and the output data for learning to the test model.

The prediction degree indicates the predictability of depressive disorder, suicide attempt or suicidal completion, or the degree to which individuals having depression or individuals who have attempted or committed suicide are distinguished from individuals not having depression or individuals not having attempted or committed suicide, when generating a test model based on the input data for learning and the output data for learning, and applying some or all of the input data for learning and the output data for learning to the test model.

After a training data set is divided into a risk group (Case) including individuals having depression or individuals who have attempted or committed suicide, and a control group including normal individuals not having depression or not having attempted or committed suicide (Control), the average of the median values, among values of the prediction degree, in the risk group and the control group, is used as a reference value for classifying the risk group and the control group. When the reference value is reapplied to the risk group and the control group in the training data set to reclassify the risk group and the control group, an algorithm and/or a method (technique), such as a method of calculating the degree of coincidence with the originally classified risk group and control group, may be used.

When machine learning is performed by including variables that have little effect on prediction of depressive disorder, suicide attempt, or suicide completion, the amount of computation may increase and the accuracy of prediction may decrease. Accordingly, in the present invention, after the test model is generated, a step (S40) is performed, in which the degree of predicting the risk of depression or suicide is obtained by applying the input data for learning and the output data for learning to the test model, and methylation-related data of which the prediction degree is greater than or equal to a predefined reference value, is selected.

The prediction degree may be about 50% or more, about 55% or more, about 60% or more, about 65% or more, about 70% or more, about 75% or more, about 80% or more, about 85% or more, about 90% or more, about 95% or more, or about 100%. According to an embodiment, the multi-omics data of which the prediction degree is 75% or more may be selected and discovered as a marker for predicting the risk of depression or suicide.

In one embodiment, the method may include the steps of: acquiring methylation-related data for a plurality of individuals having depression, a plurality of individuals who have attempted suicide, or a plurality of individuals committing suicide, and data regarding whether or not there is depression, suicide attempt or suicide completion; acquiring data regarding input data for verification, processed from the methylation-related data, and output data for verification, processed from the data regarding whether or not there is depression, suicide attempt or suicide completion; calculating the degree of replication of depressive disorder or suicide by applying the input data for verification and the output data for verification to the test model; and selecting the methylation-related data of which the replication degree is greater than or equal to a predefined reference value.

The step of acquiring methylation-related data for a plurality of individuals having depression, a plurality of individuals who have attempted suicide, or a plurality of individuals committing suicide, and data regarding whether or not there is depression, suicide attempt or suicide completion, is the same as described above. The input data for verification and the output data for verification may be acquired from the same individual from which the input data for learning and the output data for learning were acquired, or may be acquired from another individual.

Subsequently, after the step of acquiring methylation-related data and data regarding whether or not there is depression, suicide attempt or suicide completion, the step of acquiring the input data for verification and the output data for verification is performed. Data for verification may include input data for verification and output data for verification.

The "input data for verification" is processed and acquired from the methylation-related data for a plurality of individuals having depression, a plurality of individuals who have attempted suicide, or a plurality of individuals committing suicide. For example, among the methylation-related data, the values that can be classified, such as a chromosome number, the position of a nucleotide in the chromosome where methylation occurs, the degree of methylation, or the ratio of methylated sequences, may be labeled to then be converted into one mathematical value.

The "output data for verification" means data that is compared with the value output through the test model or the result value of the method for predicting the risk of depression or suicide using the same.

The output data for verification may be processed and obtained from the data regarding whether or not there is depression, suicide attempt or suicide completion. For example, the "output data for verification" may be data indicating a pathological record of being diagnosed with depressive disorder at any time in the past or in the present, an experience of a suicide attempt, or death due to suicide completion. For example, if a test model is machine-learned to predict whether or not depressive disorder, suicide attempt, or suicide completion will occur at any point in the future, the "output data for verification" may be binary data expressed as 1 for a case in which there is depression or suicide attempt or suicide completion, or expressed as 0 for a case in which there is no depressive disorder or suicide attempt or suicide completion.

After the step of acquiring the input data for verification and the output data for verification, the step of calculating the degree of replication of depressive disorder or suicide by applying the input data for verification and the output data for verification to the test model is performed.

The replication degree of depressive disorder or suicide is obtained by applying the input data for verification and the output data for verification to a pre-generated test model, thereby evaluating and verifying the performance and validity of the test model.

The replication degree indicates the predictability of depressive disorder, suicide attempt or suicidal completion, or the degree to which individuals having depression or individuals who have attempted or committed suicide are distinguished from individuals not having depression or individuals not having attempted or committed suicide, when applying some or all of the input data for verification and the output data for verification to the test model.

After a training data set is divided into a risk group (Case) including individuals having depression or individuals who have attempted or committed suicide, and a control group including normal individuals not having depression or not having attempted or committed suicide (Control), the average of the median values, among values of the replication degree, in the risk group and the control group, is used as a reference value for classifying the risk group and the control group. When the reference value is applied to the risk group and the control group in the data set for verification to classify the risk group and the control group, an algorithm and/or a method (technique), such as a method of calculating the degree of coincidence with the originally classified risk group and control group, may be used.

The replication degree may be about 50% or more, about 55% or more, about 60% or more, about 65% or more, about 70% or more, about 75% or more, about 80% or more, about 85% or more, about 90% or more, about 95% or more, or about 100% or more. According to an embodiment, the methylation-related data in which the replication degree is 50% or more may be selected and discovered as a marker for predicting the risk of depression or suicide.

In one embodiment, the method may include the steps of: acquiring psychological ideation assessment scale data for a plurality of individuals having depression, a plurality of individuals who have attempted suicide, or a plurality of individuals committing suicide; calculating a correlation between the psychological ideation assessment scale data and the methylation-related data; and selecting the methylation-related data of which the correlation is greater than or equal to a predefined reference value.

Prior to induction processing, in order to extract irrelevant or weakly related attributes, the relationship between attributes and dimensions may be analyzed. Specific attribute-related analysis methods may include information gain, Gini coefficient, uncertainty index, and correlation. The correlation means the strength of the relationship between two variables, and the existence of high correlation between the two variables may indicate that the two variables tend to increase or decrease together.

The methylation-related data may have any correlation with the psychological ideation assessment scale data. The correlation between the psychological ideation assessment scale data and the methylation-related data may be about 0.30 or more, about 0.35 or more, about 0.40 or more, about 0.45 or more, or about 0.5 or more.

According to an embodiment, the methylation-related data, between which the correlation is 0.3 or more may be selected and discovered as a marker for predicting the risk of depression or suicide.

Meanwhile, the method for discovering a marker for predicting the risk of depression or suicide using machine learning, according to an embodiment of the present invention shown in FIG. 1, can be written as a program that can be executed on a computer, and can be implemented in a general-purpose digital computer that operates the program using a computer-readable recording medium. The computer-readable recording medium may include a storage medium, such as a magnetic storage medium (e.g., a ROM, a floppy disk, a hard disk, etc.) and an optically readable medium (e.g., a CD-ROM, a DVD, etc.).

According to the method for discovering a marker for predicting the risk of depression or suicide using multi-omics analysis and machine learning according to the present invention, and an apparatus and program for performing the same, the risk of depression or suicide in an individual can be accurately predicted for each individual.

Another aspect provides a marker for predicting the risk of depression or suicide, which is discovered according to the method.

The marker for predicting the risk of depression or suicide may be methylation-related data of the 67806358th nucleotide of the 11th human chromosome, the 102516597th nucleotide of the 14th human chromosome, the 37172017th nucleotide of the 15th human chromosome, the 14014009th nucleotide of the 16th human chromosome, the 88636588th nucleotide of the 16th human chromosome, the 73009364th nucleotide of the 17th human chromosome, the 77487338th nucleotide of the 18th human chromosome, the 40023259th nucleotide of the 19th human chromosome, the 3423658th nucleotide of the second human chromosome, the 73052175th nucleotide of the second human chromosome, the 42163538th nucleotide of the 20th human chromosome, the 62460632nd nucleotide of the 20th human chromosome, the 147125005th nucleotide of the third human chromosome, the 85419584th nucleotide of the fourth human chromosome, the 21524046th nucleotide of the 6th human chromosome, or a combination thereof.

The marker for predicting the risk of depression or suicide may be methylation of the 67806358th nucleotide of the 11th human chromosome, unmethylation of the 102516597th nucleotide of the 14th human chromosome, unmethylation of the 37172017th nucleotide of the 15th human chromosome, methylation of the 14014009th nucleotide of the 16th human chromosome, methylation of the 88636588th nucleotide of the 16th human chromosome, unmethylation of the 73009364th nucleotide of the 17th human chromosome, unmethylation of the 77487338th nucleotide of the 18th human chromosome, methylation of the 40023259th nucleotide of the 19th human chromosome, unmethylation of the 3423658th nucleotide of the second human chromosome, unmethylation of the 73052175th nucleotide of the second human chromosome, unmethylation of the 42163538th nucleotide of the 20th human chromosome, unmethylation of the 62460632nd nucleotide of the 20th human chromosome, methylation of the 147125005th nucleotide of the third human chromosome, methylation of the 85419584th nucleotide of the fourth human chromosome, unmethylation of the 21524046th nucleotide of the sixth human chromosome, or a combination thereof.

The marker for predicting the risk of suicide may be methylation-related data of the 100254805th nucleotide of the 13th human chromosome, the 53093335th nucleotide of the 15th human chromosome, the 46351387th nucleotide of the 21st human chromosome, the 28390646th nucleotide of the 3rd human chromosome, the 44444362nd nucleotide of the 10th chromosome, or a combination thereof.

The marker for predicting the risk of suicide may be methylation of the 100254805th nucleotide of the 13th human chromosome, methylation of the 53093335th nucleotide of the 15th human chromosome, methylation of the 46351387th nucleotide of the 21st human chromosome, unmethylation of the 28390646th nucleotide of the third human chromosome, unmethylation of the 44144362nd nucleotide of the 10th human chromosome, or a combination thereof.

The marker for predicting the risk of suicide may specifically distinguish the risk of depression and the risk of suicide from each other. If this is applied in a reverse manner, the marker for predicting the risk of suicide can be applied as a marker for predicting the risk of depression.

Another aspect is a method for providing information for predicting the risk of depression or suicide in an individual, comprising the steps of: acquiring a nucleic acid sample from a biological sample of the individual; and analyzing methylation-related data of a marker for predicting the risk of depression or suicide from the acquired nucleic acid sample, wherein the marker is the 67806358th nucleotide of the 11th human chromosome, the 102516597th nucleotide of the 14th human chromosome, the 37172017th nucleotide of the 15th human chromosome, the 14014009th nucleotide of the 16th human chromosome, the 88636588th nucleotide of the 16th human chromosome, the 73009364th nucleotide of the 17th human chromosome, the 77487338th nucleotide of the 18th human chromosome, the 40023259th nucleotide of the 19th human chromosome, the 3423658th nucleotide of the second human chromosome, the 73052175th nucleotide of the second human chromosome, the 42163538th nucleotide of the 20th human chromosome, the 62460632nd nucleotide of the 20th human chromosome, the 147125005th nucleotide of the third human chromosome, the 85419584th nucleotide of the fourth human chromosome, the 21524046th nucleotide of the 6th human chromosome, or a combination thereof.

The method may include a step of acquiring a nucleic acid sample from a biological sample of the individual.

The individual means a subject for predicting the risk of depression or suicide. The individual may include may include vertebrates, mammals, humans (Homo sapiens), mice, rats, cattle, horses, pigs, sheep, goats, dogs, cats, and the like. For example, the human may be Asian or Korean. The terms "individual" and "subject" are used interchangeably herein.

The biological sample refers to a sample acquired from a living organism. The biological sample may be, for example, blood, tissue, urine, mucus, saliva, tears, plasma, serum, sputum, spinal fluid, pleural fluid, nipple aspirate, lymph fluid, airway fluid, intestinal fluid, genitourinary tract fluid, breast milk, lymphatic fluid, semen, cerebrospinal fluid, intratracheal fluid, ascites, cystic tumor fluid, amniotic fluid, or a combination thereof. The biological sample may contain a purely isolated nucleic acid, a coarsely isolated nucleic acid, a cell lysate containing nucleic acid, or a cell-free nucleic acid.

A method of isolating a nucleic acid from a biological sample may be performed by a conventional nucleic acid isolation method. For example, a target nucleic acid can be obtained by amplification through polymerase chain reaction (PCR), ligase chain reaction (LCR), transcription amplification, or realtime-nucleic acid (NASBA), followed by purification.

The method may include a step of analyzing the methylation-related data of a marker from the acquired nucleic acid sample. The step of analyzing the methylation-related data may be performed by a known method, by which methylation of the genome or DNA can be confirmed. For example, the step of analyzing the methylation-related data may be performed by sequencing, PCR, methylation specific PCR, real time methylation specific PCR, PCR using methylated DNA specific binding protein, quantitative PCR, DNA chip, pyrosequencing and bi sulfite sequencing, or a combination thereof.

The sequencing may be next-generation nucleotide sequencing, and "next generation sequencing (NGS)" refers to a technology in which the whole genome is fragmented in a chip-based and PCR-based paired-end format, and the fragments are subjected to sequencing at ultrahigh speed on the basis of a chemical reaction (hybridization). A large amount of sequencing data can be generated for a sample to be analyzed within a short time by the next-generation sequencing.

When the number of DNAs methylated in the marker is 1 or more, 2 or more, 3 or more, 4 or more, 5 or more, 6 or more, 7 or more, 8 or more, 9 or more, 10 or more, 11 or more, 12 or more, 13 or more, or 14 or more, it can be determined that the risk of depression or suicide is high, and the prediction accuracy can be increased.

Another aspect provides a method for predicting the risk of depression or suicide, comprising the steps of: acquiring multi-omics data for a plurality of individuals having depression, a plurality of individuals who have attempted suicide, or a plurality of individuals committing suicide, and data regarding whether or not there is depression, suicide attempt or suicide completion; generating a test model by performing machine learning on the input data for learning, processed from the multi-omics data, and the output data for learning, processed from the data regarding whether or not there is depression, suicide attempt or suicide completion; calculating the degree of predicting the risk of depression or suicide by applying the input data for learning and the output data for learning to the test model; selecting the multi-omics data of which the prediction degree is equal to or greater than a predefined reference value; and generating a model for predicting the risk of depression or suicide by using the selected multi-omics data as the input data for learning.

In one embodiment, the multi-omics data may include a method including at least one of methylation-related data and RNA expression marker data.

In one embodiment, the method for predicting the risk of depression or suicide may use a statistical prediction method or machine learning.

The predicting of the risk of depression or suicide may mean obtaining the probability of depression or suicide attempt or completion through a certain algorithm when multi-omics data including an individual's 's genetic genome, tranome, epigenome, etc., are input.

The methylation-related data are the same as described above. The RNA expression marker data may include a record related to RNA expression in the genome of an individual, such as a record regarding whether or not DNA is transcribed into RNA, as a result of sequencing within a chromosome of an individual.

The methylation-related data, the RNA expression marker data, and the data on whether or not there is depression, suicide attempt or suicide completion may be obtained from individuals in one or more hospitals or regions.

The methylation-related data may be obtained by performing a known method for confirming methylation of the genome or DNA, and can be obtained by performing a known method for confirming whether the RNA expression marker DNA is transcribed into RNA, the data regarding whether or not there is depression, suicide attempt or suicide completion may be obtained from an individual's questionnaire or survey result, but is limited thereto.

Thereafter, a test model may be generated by performing machine learning on the input data for learning, of the multi-omics data, and the output data for learning, processed from the data regarding whether or not there is depression, suicide attempt or suicide completion.

The step of generating the test model may include obtaining a correlation between multi-omics data and the output data for learning, processed from the data regarding whether or not there is depression, suicide attempt or suicide completion, corresponding to the multi-omics data, that is, mapping information of both data.

The "input data for learning" is data used for machine learning, and may be acquired by processing multi-omics data for a plurality of individuals having depression, a plurality of individuals who have attempted suicide, or a plurality of individuals committing suicide.

The multi-omics data may be processed and obtained from methylation-related data and/or RNA expression marker data. The input data for learning may include input data for first learning and/or input data for second learning. For example, among the above-described RNA expression marker data, the values that can be classified, such as a chromosome number, the position of a nucleotide in the chromosome where methylation occurs, the degree of methylation, or the ratio of methylated sequences, may be labeled to then be converted into one mathematical value.

The output data for learning means data that is compared with the value output through the test model. The output data for learning may be processed and obtained from the data regarding whether or not there is depression, suicide attempt or suicide completion. This is the same as described above.

Through this process, multi-omics data and data regarding whether or not there is depression, suicide attempt, or suicide completion can be mathematically processed to obtain input data for learning and output data for learning.

"Test model" means an input/output function that analyzes the correlation between the input data for learning and the output data for learning and diagnose depression or predicts suicide attempt, or death due to suicide completion at any point in the past, present, or future.

After the test model generation step, based on the prediction result of the test model, a step of calculating the degree of predicting the risk of depression or suicide by applying the input data for learning and the output data for learning to the test model may be performed.

The prediction degree may be the same as described above.

After generating the test model, the degree of predicting the risk of depression or suicide may be obtained by applying the input data for learning and the output data for learning to the test model, and at least one of the methylation-related data of which the prediction degree is equal to or greater than a predefined reference value, and the RNA expression marker data of which the prediction degree is equal to or greater than a predefined reference value may be selected.

The prediction degree may be about 50% or more, about 55% or more, about 60% or more, about 65% or more, about 70% or more, about 75% or more, about 80% or more, about 85% or more, about 90% or more, about 95% or more, or about 100%. According to an embodiment, the multi-omics data of which the prediction degree is 75% or more may be selected and discovered as a marker for predicting the risk of depression or suicide.

A step of generating a model for predicting the risk of depression or suicide using the selected multi-omics data as input data for learning is performed. The multi-omics data may be at least one of methylation-related data and an RNA expression marker, and in an embodiment, the result of integrating methylation-related data and/or RNA expression markers was applied to random forests, and it was confirmed from the result value that the degree for predicting the risk of depression or suicide was high.

In one embodiment, the method may include the steps of: acquiring psychological ideation assessment scale data for a plurality of individuals having depression, a plurality of individuals who have attempted suicide, or a plurality of individuals committing suicide; calculating a correlation between the psychological ideation assessment scale data and at least one of the methylation-related data and the RNA expression marker data; and selecting at least one of the methylation-related data of which the correlation is equal to or greater than a predefined reference value, and the RNA expression marker data of which the correlation is equal to or greater than a predefined reference value.

The methylation-related data and/or the RNA expression marker data may have any correlation with the psychological ideation assessment scale data. The correlation between the methylation-related data and/or the RNA expression marker data and the psychological ideation assessment scale data may be about 0.30 or more, about 0.35 or more, about 0.40 or more, about 0.45 or more, or about 0.5 or more. According to an embodiment, the methylation-related data and/or the RNA expression marker data and the psychological ideation assessment scale data, between which the correlation is 0.3 or more may be selected and finally selected as a marker for predicting the risk of depression or suicide.

In one embodiment, the step of generating the test model may include generating a test model by performing machine learning on the input data for first learning, processed from the methylation-related data, and the output data for learning, processed from the data regarding whether or not there is depression, suicide attempt or suicide completion, and modifying and updating, on the basis of the test model, a pre-generated test model by performing machine learning on the input data for second learning, processed from the RNA expression marker data, and the output data for learning, processed from the data regarding whether or not there is depression, suicide attempt or suicide completion. Thereafter, an input variable set of the modified and updated model may be selected as a final variable set, and methylation-related data of the modified and updated model, for example, may be selected as a final variable set.

In the method for discovering a maker for predicting the risk of depression or suicide and/or the method for predicting the risk of depression or suicide using a statistical prediction method or machine learning, an algorithm and/or a method (technique), such as Logistic regression, Decision tree, Nearest-neighbor classifier, Kernel discriminate analysis, Neural network, Support Vector Machine, Random forest, or Boosted tree, may be used to classify a plurality of input data for learning and/or a plurality of output data for learning.

In the method for discovering a maker for predicting the risk of depression or suicide and/or the method for predicting the risk of depression or suicide using a statistical prediction method or machine learning, an algorithm and/or a method (technique), such as Linear regression, Regression tree, Kernel regression, Support vector regression, or Deep Learning, may be used to predict the risk of depression or suicide.

In addition, in the method for discovering a maker for predicting the risk of depression or suicide and/or the method for predicting the risk of depression or suicide using a statistical prediction method or machine learning, an algorithm and/or a method (technique), such as Principal component analysis, Non-negative matrix factorization, Independent component analysis, Manifold learning, or SVD, may be used to calculate the prediction degree, the replication degree, correlation, etc.

In the method for discovering a maker for predicting the risk of depression or suicide and/or the method for predicting the risk of depression or suicide using a statistical prediction method or machine learning, an algorithm and/or a method (technique), such as k-means, Hierarchical clustering, mean-shift, or self-organizing maps (SOMs), may be used for grouping a plurality of methylation-related data.

In the method for discovering a maker for predicting the risk of depression or suicide and/or the method for predicting the risk of depression or suicide using a statistical prediction method or machine learning, an algorithm and/or a method (technique), such as Bipartite cross-matching, n-point correlation two-sample testing, or minimum spanning tree, may be used for data comparison.

However, the above-described algorithm and/or method (technique) are exemplary and the spirit of the present invention is not limited thereto.

Meanwhile, the data may be a data set. In other words, the input data for learning, the output data for learning, the input data for verification, the output data for verification, etc. may be a data set composed of a plurality of numbers (or coefficients), such as a matrix.

### ADVANTAGEOUS EFFECTS OF DISCLOSURE

According to the method for discovering a marker for predicting the risk of depression or suicide using the multi-omics analysis and machine learning of the present invention, the marker for predicting the risk of depression or suicide can be discovered with high accuracy and reliability, and the risk of depression or suicide can be diagnosed and prevented at an early stage through genetic testing. Of course, the scope of the present invention is not limited by these effects.

### BRIEF DESCRIPTION OF DRAWINGS

FIG. 1 is a flowchart illustrating a method of discovering a marker for predicting the risk of depression or suicide using multi-omics analysis and machine learning, according to an embodiment.
FIG. 2 shows a result of acquiring learning data from 70 selected subjects and analyzing the distribution of modified methyl cytosine in the entire gene.
FIG. 3 shows a process of selecting methylated sites in which the prediction and replication degrees are greater than or equal to reference values, and correlations with psychological ideation assessment scales are greater than or equal to a reference value, and DNA methylation-related data selected by the process.
FIG. 4 shows DNA methylation-related data in a group with depression and a group with suicide attempt or suicide completion.
FIG. 5 is a graph showing the degree of methylation in methylation-related data selected as a marker for predicting the risk of depression or suicide.
FIG. 6 shows a confirmation result of the degree of predicting depression or suicide from a result value obtained by applying each of a methylated site, an RNA expression result, and a result of integrating the methylated site and the RNA expression result, which are correlated with psychological ideation assessment scale data, to random forests.
FIG. 7 is a flowchart illustrating a method of discovering a marker for predicting the risk of depression or suicide using multiple omics analysis and machine learning, and a method of predicting the risk of depression or suicide using machine learning, according to an embodiment.

### MODE OF DISCLOSURE

The present invention will be described in more detail by the following examples. However, the following examples are only for helping understanding of the present invention, and the scope of the present invention is not limited by these examples in any sense.

### Example 1: 1) Extraction of genome methylation information from individuals having depression, committing suicide or attempting suicide; 2) Selection of methylated sites in which correlations with psychological ideation assessment scales are greater than or equal to reference value, and the prediction and replication degrees are greater than or equal to reference values; and 3) Prediction of the risk of depression or suicide using methylation-related data, RNA expression marker, multiple omics analysis and machine learning

### 1. Extraction of genome methylation information from individuals having depression, committing suicide or attempting suicide, and selection of methylation-related data in which the correlations with psychological ideation assessment scales are greater than or equal to a reference value, and the prediction and replication degrees are greater than or equal to reference values

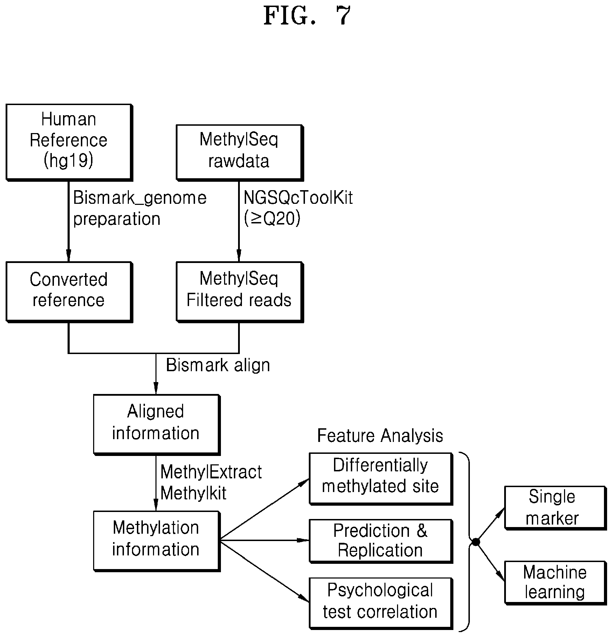

FIG. 7 is a flowchart illustrating a method for discovering a marker for predicting the risk of depression or suicide using multiple omics analysis and machine learning, and a method for predicting the risk of depression or suicide using machine learning, according to an embodiment. Referring to FIG. 7, methylseq reads acquired from individuals are aligned in the converted hg19 reference sequence, and methylation information of nucleotides is extracted. By using the above information, a marker for predicting the risk of depression or suicide may be discovered by the differentially methylated site (DMS) in each of the risk group and the normal group, the prediction and replication degrees of depression or suicide at each methylated site, and the correlation between the methylated site and the psychological ideation assessment scale, and an individual's risk of depression or suicide can be predicted using the same.

A total of 100 subjects were recruited: 22 subjects having depression, 34 subjects who attempted or committed suicide (risk group), and 44 subjects who did not attempt or commit suicide (normal group or control group). Among the recruited subjects, learning data was acquired from 70 randomly selected subjects, and verification data was acquired from the remaining 30 subjects.

Peripheral blood was collected from the 100 subjects, and then genomic DNA (gDNA) was acquired from the blood by using the QiAmp DNA kit (Qiagen, Germany). Subsequently, reduced representation bisulfite sequencing (RRBS) (Illumina) using bisulfite was performed. The acquired sequencing data was filtered by using an NGSQcToolKit to obtain only reads having a quality control of 20 or more to acquire methylseq reads. The human reference genome (hg19) was converted to a bismark_genome_preparation program. The methylseq reads were aligned to the converted hg19 reference sequence by using bismark alignment (http://genome.ucsc.edu). Methylation information was extracted from the alignment result using MethylExtract.

To compare methylation levels, sequencing samples were prepared using DNeasy Blood & Tissue Kit and Agilent SureSelectXT Human Methyl-Seq Kit 84M. Sequencing was performed through a HiSeq2500 platform. The raw data obtained by performing the sequencing was filtered using NGSQcToolKit. Alignment was performed on the filtered Methyl-seq reads for hg19 using Bismark. From the alignment result, the degree of methylation of each sample was quantified as a beta value having a value of 0 to 1 using MethylExtract. In the quantified methylation information, the effects of gender, age, and sequencing batch were removed through Combat of an SVA package. Each methylation marker was filtered through the following steps. First, the methylation position in which the methylation difference between suicide attempters and normal individuals or between patients having severe depression and normal individuals was greater than 0.01 beta value, and the Benjamini-Hochberg adjusted P value was less than 0.05 (P value < 0.05), was selected.

To compare gene expression levels, RNA-Seq samples were prepared using TruSeq RNA Sample Prep Kit v2, and sequencing was performed through HiSeq2500 platform. The raw data obtained by performing sequencing was filtered using NGSQcToolKit. The filtered RNA-seq reads were aligned to hg19 using MapSplice. From the alignment result, the gene expression of each sample was quantified using RSEM tools. In the quantified gene expression level information, the effects of gender, age, and sequencing batch were removed through Combat of an SVA package. Each gene expression marker was filtered through the following steps. First, gene expression levels between suicide attempters and normal individuals, or between patients having severe depression and normal individuals were compared using DESeq2 program. In the above analysis, the expression levels of genes in which a difference in the gene expression level is 1.2 times and the Benjamini-Hochberg adjusted P value is less than 0.05 (P-value < 0.05) were selected. Among the expression levels of the selected genes, the gene expression levels satisfying that the correlation with the psychological test score is greater than 0.2 (spearman rho > 0.2), and the P-value is less than 0.05 (P-value < 0.05), were selected once more. This means that the expression level of a gene can be significantly used as a marker for predicting the risk of suicide or depression, and can be used as an input feature set in constructing a linear regression model that can objectively score the risk of suicide or depression. By using the methylation information of 70 individuals, the differentially methylated site (DMS) in each of the risk group and the normal group was extracted using methylKit, which is a comprehensive R package for genome-wide DNA methylation profile analysis, and Wilcoxon tests.

Next, the prediction and replication degrees of suicide attempt or suicide completion at each methylated site were calculated. The prediction degree indicates the degree to which the risk group and the control group are distinguished (0 to 1) when a test model is generated using the methylation information of 70 individuals as a training data set, and the training data set is applied to the test model. The replication degree indicates the degree to which the risk group and the control group are distinguished (0 to 1) when data for verification is acquired from the remaining 30 individuals and the methylation information is applied to the generated test model. Specifically, after the training data set is divided into a risk group (Case) and a control group (Control), the average of the median values, among values of the replication degree, in the risk group and the control group, is used as a reference value for classifying the risk group and the control group. When the reference value is reapplied to the risk group and the control group of the training data set to reclassify the risk group and the control group, the value obtained by calculating the degree of coincidence with the originally classified risk group and control group may be used as the prediction degree. The value obtained by calculating the reference value in the same manner as above in the data set for verification is used as the replication degree.

In addition, based on the methylation information and the psychological ideation assessment score, the correlation between the methylated site and the psychological ideation assessment score was obtained using the Spearman correlation coefficient.

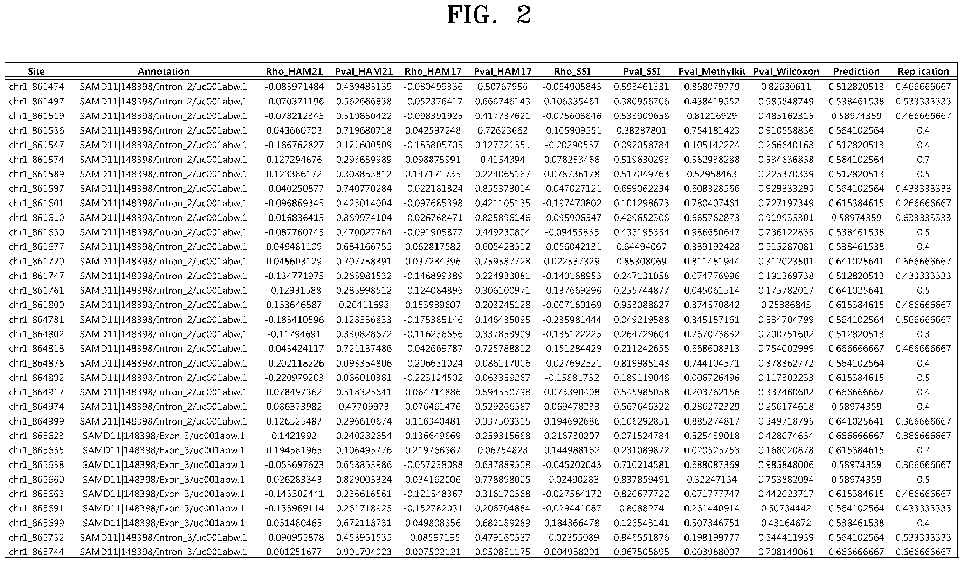

FIG. 2 shows a result of acquiring learning data from selected 70 subjects and analyzing the distribution of modified methyl cytosine in the entire gene. chr indicates a chromosome number, and Annotation indicates in which region of the gene the corresponding position is located. Rho_HAM21, HAM17, and SSI represent correlations with psychological ideation assessment scores (depression: HAM21, HAM17; suicide: SSI). Pval_HAM21, HAM17, and SSI indicate the degrees of significance of correlations with psychological ideation assessment scores. Pval_MethylKit and Pval_Willcoxon indicate significance levels of the degree to which the risk group and the control group are distinguished at each methylated site. Prediction and Replication represent a prediction degree and a degree of replication, respectively.

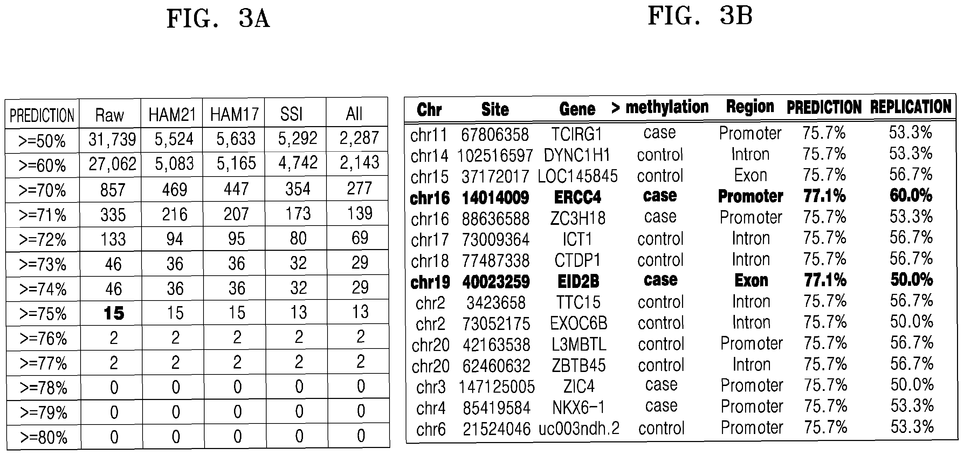

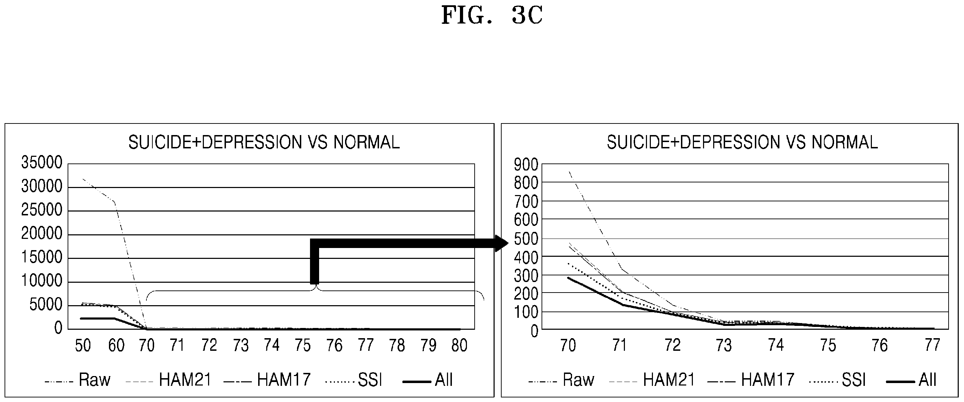

FIG. 3 shows a process of selecting methylated sites in which the prediction and replication degrees are greater than or equal to reference values, as indicated in Table of FIG. 2, and correlations with psychological ideation assessment scales are greater than or equal to a reference value, and DNA methylation related data selected by the process.

Referring to FIG. 3A, as a result of counting the methylated sites having a prediction degree of 50% or more, there are 31,739 methylated sites, among which methylated sites correlated with each psychological ideation assessment scale, were selected and counted. Here, the methylated sites in which the correlations with Rho_HAM21, HAM17, and SSI were greater than or equal to 0.3 (Rho=0.3), and the significance level of the correlation is less than 0.05 (p-value<0.05) were selected as the associated methylated sites. As a result, the selected associated methylated sites were 5,524, 5,633, and 5,292 for HAM21, HAM17, and SSI, respectively. The number of the methylated sites correlated with all psychological ideation assessment scale was 2,287.

Among the associated methylated sites, 15 methylated sites in which the prediction degree is 75% or more were selected and shown in FIG. 3B. As shown in FIG. 3B, the 15 kinds of methylation-related data enable the risk of suicide attempt or suicide completion, or depression to be predicted with high accuracy and reliability. In FIG. 3B, chr indicates a chromosome number, site indicates a position on the chromosome, gene indicates which gene the corresponding position is correlated with, >methylation indicates which group is more methylated between the risk group and the normal group at the corresponding position, and region indicates in which region of the gene the corresponding position is located. FIG. 3C is a graphical representation of FIGS. 3A and 3B.

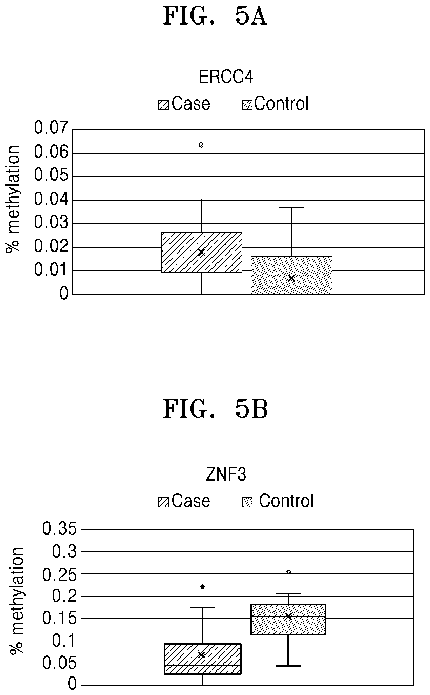

FIG. 5 is a graph showing the degree of methylation in the methylation-related data selected as a marker for predicting the risk of depression or suicide. FIG. 5A is a graph showing the degree of methylation with respect to the 14014009th nucleotide of the 16th human chromosome, which is a methylated site, in individuals having depression or individuals who have attempted suicide or committing suicide. As shown in FIG. 5A, the individuals having depression or individuals who have attempted suicide or committing suicide had a significantly high degree of methylation at the 14014009th nucleotide of the 16th human chromosome, compared to the normal group.

### 2. Selection of methylated sites specifically associated with suicide completion or suicide attempt

Since the risk of depression and suicide attempt or suicide completion can be induced by other genetic factors, methylation-related data that can distinguish depression from suicide attempt or suicide completion was additionally identified in the same manner as in Section 1.

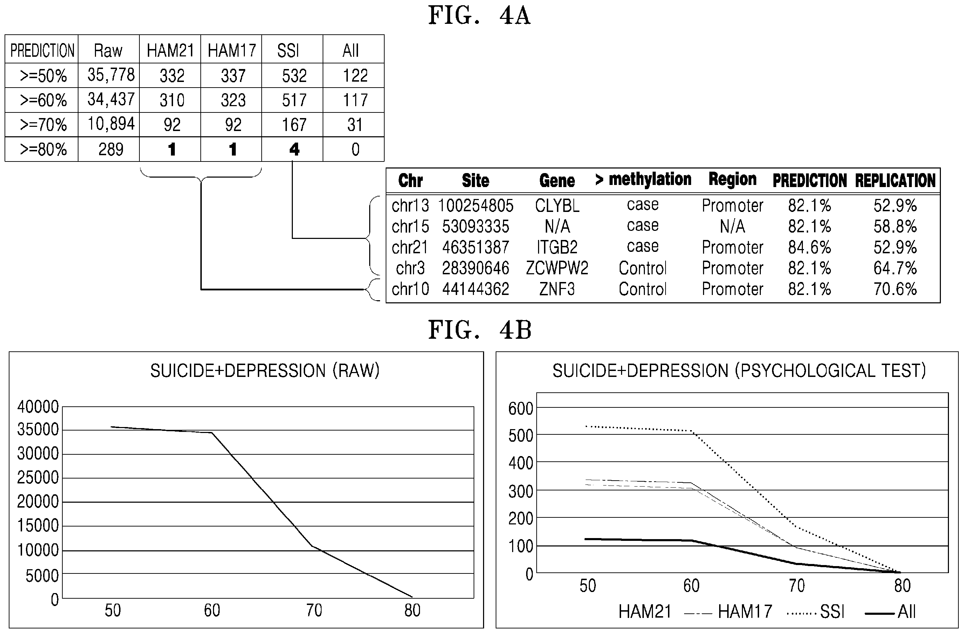

FIG. 4 shows DNA methylation-related data in a group with depression and a group with suicide attempt or suicide completion.

Referring to FIG. 4A, as a result of counting methylated sites in which the degree of predicting the risk of suicide attempt or suicide completion is greater than or equal to 50%, the number of counted methylated sites was 35,778, among which the methylated sites correlated with each psychological ideation assessment scale were selected and counted. As a result, the selected associated methylated sites were 322, 337, and 532 for HAM21, HAM17, and SSI, respectively. The number of the methylated sites correlated with all psychological ideation assessment scale was 122. Among the associated methylated sites, the number of the methylated sites in which the prediction degree is 80% or more and which are correlated with each psychological ideation assessment scale, was 5. As shown in FIG. 4A, the kind of methylation-related data enable the risk of suicide attempt or suicide completion, or depression to be predicted with high accuracy and reliability by specifically discriminating the risk of suicidal ideation or suicide attempt from the risk of depression. FIG. 4B is a graphical representation of FIG. 4A.

FIG. 5 is a graph showing the degree of methylation in the methylation-related data selected as a marker for predicting the risk of depression or suicide. FIG. 5B is a graph showing the degree of methylation in the group having depression and in the group attempting suicide or committing suicide with respect to the 44444362nd nucleotide of the 10th human chromosome, which is a methylated site. As shown in FIG. 5B, the individuals having depression had a significantly high degree of methylation at the 44144362nd nucleotide of the 10th human chromosome, compared to the individuals who have attempted suicide or committing suicide. Meanwhile, it can be seen that the individuals who have attempted suicide or committing suicide have methylation of the 100254805th nucleotide of the 13th human chromosome, methylation of the 53093335th nucleotide of the 15th human chromosome, methylation of the 46351387th nucleotide of the 21st human chromosome, unmethylation of the 28390646th nucleotide of the third human chromosome, and unmethylation of the 44144362nd nucleotide of the 10th human chromosome.

### 3. Prediction of the risk of depression or suicide using methylation-related data, RNA expression marker, multiple omics analysis and machine learning

The methylated sites (86 sites) correlated with three kinds of psychological ideation assessment scales (with correlation of 0.35 or more) were used and applied to random forests, one of the machine learning methods. Since the results for the risk group having the risk of depression or suicide and the normal group were confirmed in Section 1, the degree of predicting the risk of depression or suicide was confirmed by applying a supervised learning method. For validation, among various validation methods, a leaveone-out cross validation method which is useful for a small number of samples was applied.

The methylation sites, the multi-omics analysis and the method for discovering a marker for predicting the risk of depression or suicide using machine learning, which were performed in Section 1, were applied to RNA expression data. In addition, the RNA expression data (28 pieces) correlated with three kinds of psychological ideation assessment scales (with correlation of 0.35 or more) were applied to supervised random forests.

The methylation sites, the RNA expression data, and Wilcoxon signed-rank test results were used and applied to supervised random forests.

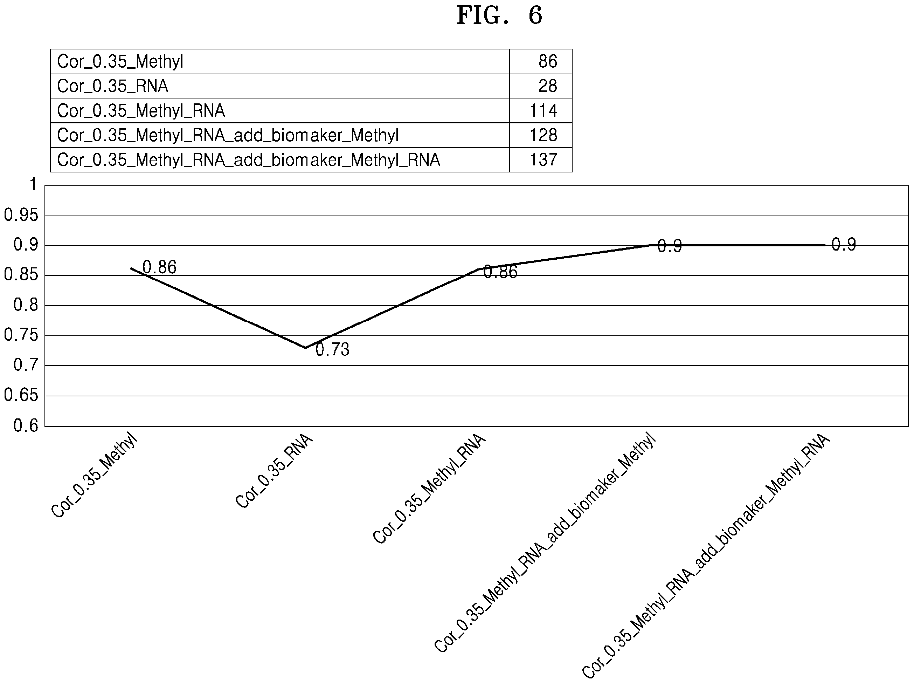

FIG. 6 shows a confirmation result of the degree of predicting depression or suicide from a result value obtained by applying each of the methylated site, the RNA expression result, and the result of integrating the methylated site and the RNA expression result, which are correlated with the psychological ideation assessment scale data, to random forests.

Referring to FIG. 6, the accuracy of predicting the risk of depression or suicide for the methylation sites (86 sites), which are correlated with the three kinds of psychological ideation assessment scales was about 86%. The accuracy of predicting the risk of depression or suicide for the RNA expression results, which are correlated with the three kinds of psychological ideation assessment scales was about 73%. The accuracy of predicting the risk of depression or suicide for the integrated data (114 pieces) of the methylated sites and the RNA expression results, which are correlated with the three kinds of psychological ideation assessment scales, was about 86%. When 15 kinds of markers analyzed and confirmed in Section 1 were added to the integrated data (114 pieces) of the methylated sites and the RNA expression results, which are correlated with the three psychological ideation assessment scales, the accuracy of predicting the risk of depression or suicide was about 90%. When 15 kinds of markers analyzed and confirmed in Section 1 and 9 kinds of RNA expression markers were added to the integrated data (114 pieces) of the methylated sites and the RNA expression results, which are correlated with the three psychological ideation assessment scales, the accuracy of predicting the risk of depression or suicide was about 90%.

The risk of depression or suicide in an individual can be predicted with high accuracy through a certain algorithm and multi-omics data including the individual's tranome, epigenome, etc.

## Claims

1. A method for discovering a marker for predicting a risk of depression or suicide, the method comprising:
acquiring multi-omics data for a plurality of individuals having depression, a plurality of individuals who have attempted suicide, or a plurality of individuals who have committed suicide, and data regarding whether or not there is depression, suicide attempts or suicide completion;
generating a test model by performing machine learning on the input data for learning, processed from the multi-omics data, and the output data for learning, processed from the data regarding whether or not there is depression, suicide attempts or suicide completion;
calculating a degree of predicting the risk of depression or suicide, by applying the input data for learning and the output data for learning to the test model; and
selecting the multi-omics data of which the degree of prediction is equal to or greater than a predefined reference value.

2. The method of claim 1, wherein the multi-omics data includes methylation-related data or genome data.

3. The method of claim 2, wherein the methylation-related data or genome data includes a change in a measured methylation level or a measured gene expression level, compared to a methylation level or a gene expression level of a comparative control group.

4. The method of one of claims 1 to 3, wherein the method of predicting the risk of depression or suicide uses machine learning.

5. The method of claim 4, comprising: acquiring multi-omics data for a plurality of individuals having depression, a plurality of individuals who have attempted suicide, or a plurality of individuals who have committed suicide, and data regarding whether or not there is depression, suicide attempts or suicide completion;
acquiring data regarding input data for verification, processed from the multi-omics data, and output data for verification, processed from the data regarding whether or not there is depression, suicide attempts or suicide completion;
calculating a degree of replication of depression or suicide by applying the input data for verification and the output data for verification to the test model; and
selecting the methylation-related data of which the degree of replication is greater than or equal to a predefined reference value.

6. The method of claim 4, comprising: acquiring psychological ideation assessment scale data for a plurality of individuals having depression, a plurality of individuals that have attempted suicide, or a plurality of individuals that have committed suicide;
calculating a correlation between the psychological ideation assessment scale data and the methylation-related data; and
selecting the methylation-related data of which the correlation is greater than or equal to a predefined reference value.

7. The method of claim 4, wherein the reference value for the degree of prediction is 50%

8. The method of claim 5, wherein the reference value for the degree of replication is 50%.

9. The method of claim 6, wherein the reference value for the correlation is 0.3.

10. A marker for predicting a risk of depression or suicide, discovered by the method of one of claims 1 to 3.

11. A marker for predicting a risk of depression or suicide, discovered by the method of claim 4.

12. A marker for predicting a risk of depression or suicide, wherein the marker is methylation-related data of the 67806358th nucleotide of the 11th human chromosome, the 102516597th nucleotide of the 14th human chromosome, the 37172017th nucleotide of the 15th human chromosome, the 14014009th nucleotide of the 16th human chromosome, the 88636588th nucleotide of the 16th human chromosome, the 73009364th nucleotide of the 17th human chromosome, the 77487338th nucleotide of the 18th human chromosome, the 40023259th nucleotide of the 19th human chromosome, the 3423658th nucleotide of the second human chromosome, the 73052175th nucleotide of the second human chromosome, the 42163538th nucleotide of the 20th human chromosome, the 62460632nd nucleotide of the 20th human chromosome, the 147125005th nucleotide of the third human chromosome, the 85419584th nucleotide of the fourth human chromosome, the 21524046th nucleotide of the 6th human chromosome, or a combination thereof.

13. A method of providing information for predicting a risk of depression or suicide in an individual, comprising:
acquiring a nucleic acid sample from a biological sample of the individual; and
analyzing methylation-related data of a marker for predicting the risk of depression or suicide from the acquired nucleic acid sample, wherein
the marker is methylation-related data of the 67806358th nucleotide of the 11th human chromosome, the 102516597th nucleotide of the 14th human chromosome, the 37172017th nucleotide of the 15th human chromosome, the 14014009th nucleotide of the 16th human chromosome, the 88636588th nucleotide of the 16th human chromosome, the 73009364th nucleotide of the 17th human chromosome, the 77487338th nucleotide of the 18th human chromosome, the 40023259th nucleotide of the 19th human chromosome, the 3423658th nucleotide of the second human chromosome, the 73052175th nucleotide of the second human chromosome, the 42163538th nucleotide of the 20th human chromosome, the 62460632nd nucleotide of the 20th human chromosome, the 147125005th nucleotide of the third human chromosome, the 85419584th nucleotide of the fourth human chromosome, the 21524046th nucleotide of the 6th human chromosome, or a combination thereof.

14. A method of predicting a risk of depression or suicide, comprising:
acquiring multi-omics data for a plurality of individuals having depression, a plurality of individuals who have attempted suicide, or a plurality of individuals who have committed suicide, and data regarding whether or not there is depression, suicide attempts or suicide completion;
generating a test model by performing machine learning on the input data for learning, processed from the multi-omics data, and the output data for learning, processed from the data regarding whether or not there is depression, suicide attempt or suicide completion;
calculating a degree of predicting the risk of depression or suicide by applying the input data for learning and the output data for learning to the test model;
selecting the multi-omics data of which the degree of prediction is equal to or greater than a predefined reference value; and
generating a model for predicting the risk of depression or suicide by using the selected multi-omics data as the input data for learning.

15. The method of claim 14, wherein the multi-omics data includes at least one of methylation-related data and RNA expression marker data.

16. The method of claim 14 or 15, wherein the method uses a statistical prediction method or machine learning.

17. The method of claim 16, comprising: acquiring psychological ideation assessment scale data for a plurality of individuals having depression, a plurality of individuals who have attempted suicide, or a plurality of individuals who have committed suicide;
calculating a correlation between the psychological ideation assessment scale data and at least one of the methylation-related data and the RNA expression marker data; and
selecting at least one of the methylation-related data of which the correlation is greater than or equal to a predefined reference value and the RNA expression marker data of which the correlation is greater than or equal to a predefined reference value.

18. The method of claim 16, wherein the generating of a test model comprises:
generating a test model by performing machine learning on the input data for first learning, processed from the methylation-related data, and the output data for learning, processed from the data regarding whether or not there is depression, suicide attempts or suicide completion, and
updating, on the basis of the test model, a pre-generated test model by performing machine learning on the input data for second learning, processed from the RNA expression marker data, and the output data for learning, processed from the data regarding whether or not there is depression, suicide attempts or suicide completion.
